# EUROPEAN PATENT APPLICATION

(11) **EP 2 078 526 A1**
(43) Date of publication of application: **15.07.2009**
(21) Application number: 07829584.7
(22) Date of filing: 11.10.2007
(51) Int. Cl.: A61K 31/496, A61K 9/08, A61K 47/02, A61K 47/36, A61P 27/02, A61P 31/04

(54) **AQUEOUS LIQUID PREPARATION HAVING IMPROVED INTRAOCULAR GATIFLOXACIN PENETRATION**

(30) Priority: 12.10.2006 JP 2006279007
(71) Applicant: Kyorin Pharmaceutical Co., Ltd., Tokyo 101-8311 (JP); Senju Pharmaceutical Co., Ltd., Osaka-shi, Osaka 541-0046 (JP)
(72) Inventor: SAWA, Shirou, Kobe-shi Hyogo 651-2241 (JP)
(74) Representative: Gillard, Richard Edward
(86) International application number: PCT/JP2007/069846
(87) International publication number: WO 2008/044733

(57) **Abstract**

An object is to provide an ophthalmic aqueous preparation excellent in the retention of gatifloxacin in a tear fluid and the preparation of gatifloxacin into an aqueous humor and a conjunctiva. Another object is to prevent the formation of any precipitate and the reduction in viscosity in the aqueous liquid preparation. An aqueous liquid preparation comprising gatifloxacin, a pharmacologically acceptable salt thereof or a hydrate of gatifloxacin or the salt and at least 0.15 w/v% of xanthan gum enables to improve the retention and penetration of gatifloxacin. When at least 0.2 w/v% of sodium chloride is added to the aqueous liquid preparation, the formation of any precipitate and the reduction in viscosity in the aqueous liquid preparation can be prevented.

## Description

### Technical Field

The present invention relates to an aqueous liquid preparation having improved intraocular penetration of gatifloxacin (chemical name: (+)-1-cyclopropyl-6-fluoro-1,4-dihydro-8-methoxy-7-(3-methyl-1-piperazinyl)-4-oxo-3-quinolinecarboxylic acid). More specifically, it relates to an opthalmic aqueous liquid preparation in which intraocular penetration of gatifloxacin is improved by xanthan gum.

### Background Art

Gatifloxacin is a quinolonecarboxylic acid derivative and is a new quinolone synthetic antibacterial agent. It is recognized to exhibit strong antibacterial potency against not only Gram-negative bacteria but also Gram-positive bacteria, anaerobic bacteria, and mycoplasma, and an application to infectious diseases in ophthalmological area such as conjunctivitis, dacryoadenitis and hordeolum, and infectious diseases in otological area such as otitis externa, tympanitis and sinusitis has been proposed (see Patent Document 1). In case of antibacterial agent-containing eye drops, exacerbation of corneal permeability of a drug, and increase in an amount of penetration into an aqueous humor become an index of preparation design.

However, generally, a drug applied to eyes is hardly penetrated into eyes due to dilution with a tear fluid and the barrier function of a cornea. For this reason, it is necessary to improve intraocular penetration of a drug, and design a preparation so as to enhance the effect of a drug. Regarding gatifloxacin, Patent Document 2 discloses an aqueous liquid preparation having raising corneal penetration of gatifloxacin, containing gatifloxacin or its salt and sodium edetate. In addition, this publication also discloses a method for preventing precipitation of a crystal of gatifloxacin.

On the other hand, regarding use of xanthan gum in a pharmaceutical composition, Patent Document 3 discloses an aqueous composition containing an ophthalmic drug and 0.01 to 2.5% xanthan gum, and describes that xanthan gum enhances the therapeutic effect of a cholinergic drug such as Echothiopate. In addition, its Example 2 describes an eye drop formulation containing Pilocarpine, xanthan gum and 0.04% of potassium chloride. Patent Document 4 describes a liquid pharmaceutical suspension containing xanthan gum, in which bitterness of a pharmaceutically active drug such as a quinolonecarboxylic acid antibiotic or the like is masked. Patent Document 5 discloses a pharmaceutical composition containing a fluoroquinolone antibiotic and xanthan gum, and describes that a pharmaceutical composition which becomes a grade (NTU) having a turbidity of 40 or less at room temperature can be obtained by adding at least 0.15% (W/W) of a water-soluble calcium salt to a water-soluble pharmaceutical composition containing xanthan gum (0.4 to 0.8% (W/W)) and the fluoroquinolone antibiotic. Patent Document 6 discloses an ophthalmic composition having a total ionic intensity of about 120 mM or less, which is gelled upon contact with eyes. This invention is based on the finding that the strength of a gel formed by xanthan gum upon contact with lysozyme depends on an original content of bonded acetate and an original content of pyruvic acid salt of xanthan gum in a composition, and as an ophthalmic drug, quinolones (e.g. ciprofloxiacin) and amino glycoside are exemplified as an anti-infectious agent.
Patent Document 1: JP-8-9597 B
Patent Document 2: WO 00/10570 A
Patent Document 3: US Patent No.4136177
Patent Document 4: JP 2004-535370 A
Patent Document 5: JP 2003-513046 A
Patent Document 6: JP 2002-510654 A

### Disclosure of the Invention

### Problem to be Solved by the Invention

A main object of the present invention is to provide an aqueous liquid preparation excellent in intraocular penetration of gatifloxacin, that is, excellent in retention in a tear fluid, and penetration into an aqueous humor and a conjunctiva of gatifloxacin.

As described above, in order to achieve this object, in the present invention, xanthan gum is used, but an aqueous liquid preparation containing gatifloxacin and xanthan gum causes formation of a precipitate and reduction in viscosity in some cases. Therefore, another object of the present invention to provide an aqueous liquid preparation in which the formation of a precipitate and the reduction in viscosity are suppressed.

### Means for Solving the Problem

The present inventor has intensively studied so as to achieve these objects and, as a result, has found that intraocular penetration of gatifloxacin can be improved by incorporating xanthan gum together with gatifloxacin or its pharmacologically acceptable salt, or a hydrate thereof into a preparation. Further, it has been found that the object of suppressing the formation of a precipitate and the reduction in viscosity is overcome by adding sodium chloride.

The present invention has been completed based on these findings, and provides:
(1) An aqueous liquid preparation comprising gatifloxacin or its pharmacologically acceptable salt, or a hydrate thereof, at least 0.15 w/v% of xanthan gum and at least 0.2 w/v% of sodium chloride, and having a pH of 5 to 8;
(2) The aqueous liquid preparation according to the above (1), wherein the preparation contains 0.15 to 1.0 w/v% of xanthan gum;
(3) The aqueous liquid preparation according to the above (1), wherein the preparation contains 0.2 to 1.8 w/v% of sodium chloride;
(4) The aqueous liquid preparation according to the above (1), wherein the preparation contains 0.1 to 1.0 w/v% of gatifloxacin;
(5) The aqueous liquid preparation according to any one of the above (1) to (4), which is an eye drop;
(6) A method for improving intraocular penetration of gatifloxacin, which comprises incorporating at least 0.15 w/v% of xanthan gum into an aqueous liquid preparation containing gatifloxacin or its pharmacologically acceptable salt thereof, or a hydrate thereof;
(7) A method for suppressing formation of a precipitate and reduction in viscosity in an aqueous liquid preparation containing gatifloxacin and xanthan gum, which comprises incorporating at least 0.2 w/v% of sodium chloride into an aqueous liquid preparation containing gatifloxacin or its pharmacologically acceptable salt thereof, or a hydrate thereof, and at least 0.15 w/v% of xanthan gum; and the like.

### Effect of the Invention

According to the present invention, intraocular penetration of gatifloxacin is improved by incorporating a predetermined amount of xanthan gum into in an aqueous liquid preparation containing gatifloxacin, its pharmacologically acceptable salt or a hydrate thereof. In addition, the formation of a precipitate and the reduction in viscosity in an aqueous liquid preparation containing gatifloxacin and xanthan gum can be suppressed by incorporating a predetermined amount of sodium chloride into an aqueous liquid preparation containing gatifloxacin, its pharmacologically acceptable salt, or a hydrate thereof, and xanthan gum.

### Brief Description of Drawings

Fig. 1 is a graph showing results of measurement of gatifloxacin concentration in a tear fluid in Test Example 1.
Fig. 2 is a graph showing results of measurement of gatifloxacin concentration in an aqueous humor in Test Example 1.
Fig. 3 is a graph showing results of measurement of gatifloxacin concentration in a conjunctiva in Test Example 1.

### Best Mode for Carrying Out the Invention

In the present specification, improvement in intraocular penetration means improvement in an amount of penetration of a drug into a tear fluid, an aqueous humor or a conjunctiva.

The present invention includes gatifloxacin, its pharmacologically acceptable salt, or a hydrate thereof as an active ingredient. Examples of the pharmacologically acceptable salt of gatifloxacin include salts with inorganic acids such as hydrochloric acid, sulfuric acid and phosphoric acid, salts with organic acids such as methanesulfonic acid, lactic acid, oxalic acid and acetic acid, and salts of sodium, potassium, magnesium, calcium, aluminum, cerium, chromium, cobalt, copper, iron, zinc, platinum, silver and the like. Examples of the hydrate include 2/5, 1/2, 3/2, and 5 hydrates.

The amount of gatifloxacin, its pharmacologically acceptable salt, or a hydrate thereof to be incorporated into the aqueous liquid preparation of the present invention is varied depending on a degree of infection to be subjected, but the amount is usually 0.1 to 1.0 w/v%, and preferably 0.2 to 0.8 w/v% as free gatifloxacin based on the total amount of the aqueous liquid preparation.

The average molecular weight of xanthan gum used in the aqueous liquid preparation of the present invention is usually 100000 to 50000000, preferably 200000 to 20000000, and particularly preferably 1000000 to 10000000. Examples of the xanthan gum to be used include Echo Gum Series such as Echo Gum T and Echo Gum F commercially available from Dainippon Sumitomo Pharma Co., Ltd., San Ace Series such as San Ace NXG-S commercially available from San Ei Gen F.F. I., Inc., Keltrol Series such as Keltrol CG and Keltrol CG-T commercially available from Sansho Co. Ltd., and the like. Preferable are Echo Gum T and Keltrol CG-T.

The amount of xanthan gum to be incorporated into the preparation is 0.15 to 1.0 w/v%, preferably 0.2 to 0.5 w/v% based on the total amount of the aqueous liquid preparation.

Viscosity of the aqueous liquid preparation of the present invention is usually about 15 to 150 mPa·s at 20 °C, and the amount of sodium chloride to be incorporated into the preparation for suppressing formation of a precipitate, and suppressing reduction in viscosity is at least 0.2 w/v% or more, preferably 0.2 to 1.8 w/v%, more preferably 0.2 to 1.5 w/v%, and further preferably 0.7 to 1.0 w/v% based on the total amount of the aqueous liquid preparation. For example, the amount of sodium chloride to be incorporated for suppressing formation of a precipitate is at least 0.2 w/v% or more, preferably 0.2 to 1.8 w/v%, and more preferably 0.2 to 1.0 w/v% based on the total amount of the aqueous liquid preparation. The amount of sodium chloride to be incorporated for suppressing reduction in viscosity is at least 0.2 w/v% or more, preferably 0.2 to 1.8 w/v%, more preferably 0.5 to 1.5 w/v%, and further preferably 0.7 to 1.0 w/v% based on the total amount of the aqueous liquid preparation.

The aqueous liquid preparation of the present invention can be administered orally or parenterally. Examples of a dosage form include oral dosage forms such as syrups and parenteral dosage forms such as solution-like injectable preparations, external preparations such as eye drops, etc., and the aqueous liquid preparation is preferably used in a form for ocular topical administration and particularly preferably eye drops.

The pH of the aqueous liquid preparation of the present invention is usually 5 to 8, preferably 5.5 to 7.5, further preferably 5.8 to 7.2, and particularly preferably 6 to 7.

Further, if necessary, the aqueous liquid preparation of the present invention may appropriately contain isotonicities (e.g. potassium chloride, boric acid, glycerin, propylene glycol, mannitol, sorbitol, glucose, etc.), buffers (e.g. phosphate buffer, acetate buffer, borate buffer, citrate buffer, glutamic acid, ε-aminocaproic acid, etc.), preservatives (benzalkonium chloride, benzethonium chloride, chlorohexidine gluconate, chlorobutanol, benzyl alcohol, sodium dehydroacetate, paraoxybenzoic acid esters, etc.), thickeners (methylcellulose, hydroxyethylcellulose, hydroxypropylmethylcellulose, carboxymethylcellulose, sodium hyaluronate, carboxyvinyl polymer, polyvinyl alcohol, polyvinylpyrrolidone, macrogol, etc.), pH adjusting agents (hydrochloric acid, sodium hydroxide, acetic acid, phosphoric acid etc.), stabilizers (sodium edetate, citric acid, etc.) and the like.

The aqueous liquid preparation of the present invention may be produced by a per se known method, and can be produced, for example, by the method described in 15^{th} revision Japanese Pharmacopoeia, Preparation General Rule, Eye drops or Liquid Preparations.

The aqueous liquid preparation of the present invention, for example, eye drops have antibacterial activity, and can be applied one drop to each eye 1 to 3 times a day for preventing or treating blepharitis, hordeolum, dacryocytitis, conjunctivitis, inflammation of the tarsal gland, keratitis, cornea ulcer, post-operation infectious disease, and the like. For otitis externa and tympanitis, the preparation can be usually applied 6 to 10 drops into each ear 1 to 2 times a day. In addition, for sinusitis, usually, the preparation can be spray-inhaled 2 to 4 ml every other day three times per week, or the preparation can be injected into maxillary sinus 1 ml per week. In this regard, the number of applications can be appropriately increased or decreased depending on a degree of a particular symptom.

### Examples

The following Examples and Test Examples will further illustrate the present invention in detail, but the present invention is not limited thereto.

### Example 1 and Comparative Examples 1 to 2

According to the formulations of Table 1, gatifloxacin-containing eye drops were prepared by a conventional method.

**Table 1**

| Formulation | Comparative Example 1 | Comparative Example 2 | Example 1 |
|---|---|---|---|
| Gatifloxacin 3/2 hydrate | 0.32 g | 0.32 g | 0.32 g |
| Sodium chloride | 0.96 g | 0.96 g | 0.96 g |
| Xanthan gum | - | 0.1 g | 0.2 g |
| Hydrochloric acid | adequate amount | adequate amount | adequate amount |
| Purified water | adequate amount | adequate amount | adequate amount |
| Total amount | 100 mL | 100 mL | 100 mL |
| pH | 6.0 | 6.0 | 6.0 |

As xanthan gum, Echo Gum T manufactured by Dai Nippon Sumitomo Pharma Co., Ltd. was used.

### Test Example 1

Effect of xanthan gum on retention in a tear fluid, and penetration into an aqueous humor and a conjunctiva of gatifloxacin

### 1. Experimental Method

### (1) Gatifloxacin concentration in tear fluid

### (a) Administration

Each 50 µL of eye drops of Table 1 (Comparative Examples 1 and 2, Example 1) was applied to one eye of a rabbit (weight 2.20 to 2.71 kg).

### (b) Method of collecting tear fluid

After 5, 15, 30 and 60 minutes of instillation, a tear fluid was collected using a capillary (MICROCAPS 2µL, Drummond).

### (c) Method of preparing sample solution

A weight of the collected tear fluid was measured, 0.3 mL of a mobile phase for HPLC was added to dilute the tear fluid, which was used as a sample solution, and a gatifloxacin concentration was measured by the HPLC method.

### (2) Gatifloxacin concentration in aqueous humor and conjunctiva

### (a) Administration

Each 50 µL of eye drops (Comparative Example 1, Example 1) of Table 1 were applied to one eye of a rabbit (weight 2.20 to 2.71 kg).

### (b) Method of collecting aqueous humor and conjunctiva

After 1 and 2 hours of instillation, an excess amount of 5% pentobarbital sodium was administered to euthanize the animals. After an anterior eye was washed with physiological saline, an aqueous humor and a conjunctiva were collected. The aqueous humor was collected with a syringe with a 27 G injection needle, and the conjunctiva was directly cut with scissors.

### (c) Method of preparing sample solution

The aqueous humor was filtered with a 0.22 µm membrane filter, which was used as a sample solution. The conjunctiva, after measurement of the weight, was finely cut with addition of 5 mL of acetonitrile, followed by shaking (200 rpm × 20 min) and centrifugation (2000 rpm × 10 min). Four milliliter of this supernatant was taken, evaporated to dryness under reduced pressure, and then this was dissolved in 0.5 mL of a mobile phase for HPLC, and filtered with a filter (0.22 µm), which was used as a sample solution, and gatifloxacin concentration was measured by the HPLC method.

### 2. HPLC analysis conditions

Detector: Ultraviolet visible spectrophotometer
(measurement wavelength: 280 nm)
Column: Inertsil ODS-3 4.6 mmφ × 150 mm, 5 µm (GL Sciences Inc.)
Guard column: Inertsil ODS-3 4.6 mmφ × 5 mm (GL Sciences Inc.)
Column temperature: 40°C
Mobile phase: Phosphoric acid was added to a mixture of acetonitrile, distilled water and triethylamine (18 : 81 : 1) to adjust a pH to 4.5.
Flow rate: 0.8 mL/min
Injection amount: 50 µL
Setting temperature of sample cooler: 4°C

### 3. Results (data)

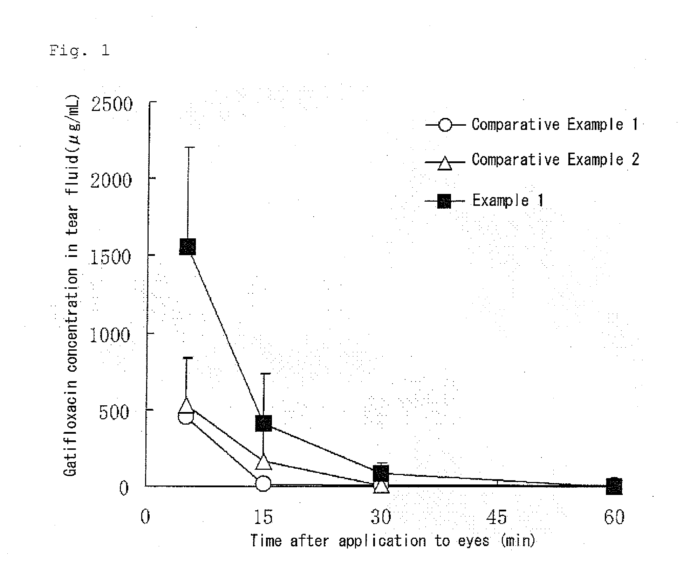

Gatifloxacin concentration (n=6) in a tear fluid is shown in Table 2 and Fig. 1.

**Table 2**

| Time | Comparative Example 1 | Comparative Example 2 | Example 1 |
|---|---|---|---|
| 5 min | 452.1±387.4 | 535.9±290.4 | 1555.8±.645.2 |
| 15 min | 17.4±16.8 | 164.4±223.1 | 411.2±322.1 |
| 30 min | 5.20±3.10 | 11.20±6.40 | 91.0±64.7 |
| 60 min | 6.70±13.70 | 0.80±0.40 | 2.80±2.00 |

| | | | |
|---|---|---|---|
| Each value indicates average ± standard deviation (µg/mL) | | | |

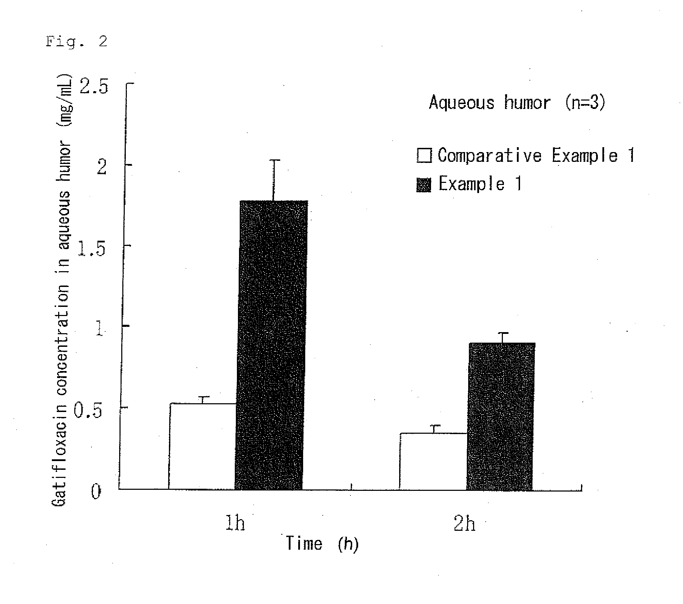

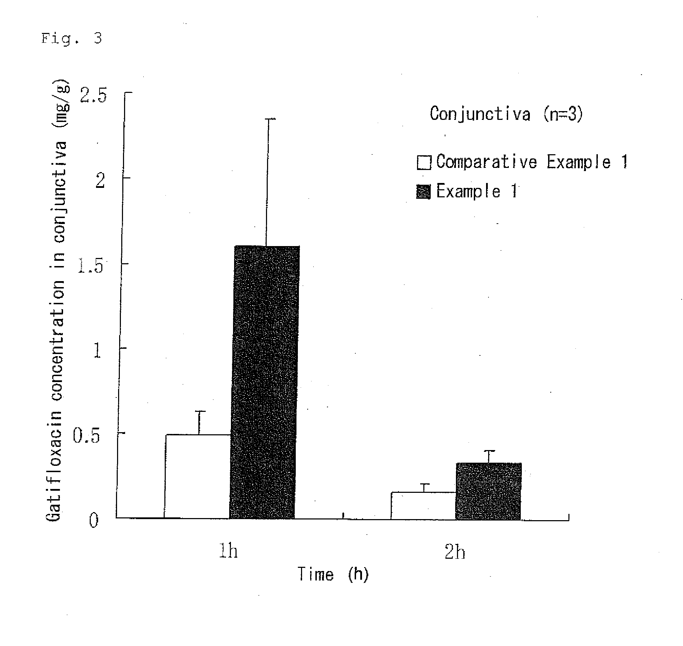

Gatifloxacin concentration (n=3) in an aqueous humor and a conjunctiva is shown in Table 3, Fig. 2 and Fig. 3.

**Table 3**

| | | Aqueous humor | | Conjunctiva | |
|---|---|---|---|---|---|
| Formulation | | Comparative Example 1 | Example 1 | Comparative Example 1 | Example 1 |
| Time | 1h | 0.525±0.045 | 1.779±0.251 | 0.490±0.141 | 1.603±0.752 |
| | 2h | 0.351±0.049 | 0.904±0.063 | 0.165+0.049 | 0.335±0.076 |

| | | | | | |
|---|---|---|---|---|---|
| Each value indicates average ± standard deviation (aqueous humor µg/ml, conjunctiva µg/g) | | | | | |

### Examples 2 to 9 and Comparative Examples 3 to 12

According to the formulations of Tables 4 to 5, gatifloxacin-containing eye drops were prepared by a conventional method.

**Table 4**

| Formulation (g/100 mL) | Comparative Example 3 | Comparative Example 4 | Comparative Example 5 | Comparative Example 6 | Comparative Example 7 | Example 2 | Example 3 | Example 4 |
|---|---|---|---|---|---|---|---|---|
| Gatifloxacin 3/2 hydrate | - | - | - | - | 0.32 | 0.32 | 0.32 | 0.32 |
| Xanthan gum | 0.2 | 0.2 | 0.2 | 0.2 | 0.2 | 0.2 | 0.2 | 0.2 |
| Sodium chloride | - | 0.2 | 0.7 | 0.9 | - | 0.2 | 0.7 | 0.9 |
| Hydrochloric acid | adequate amount | adequate amount | adequate amount | adequate amount | adequate amount | adequate amount | adequate amount | adequate amount |
| Sodium hydroxide | adequate amount | adequate amount | adequate amount | adequate amount | adequate amount | adequate amount | adequate amount | adequate amount |
| Purified water | adequate amount | adequate amount | adequate amount | adequate amount | adequate amount | adequate amount | adequate amount | adequate amount |
| Total amount | 100 mL | 100 mL | 100 mL | 100 mL | 100 mL | 100 mL | 100 mL | 100 mL |
| pH | 6.1 | 5.5 | 5.3 | 5.3 | 6.0 | 6.0 | 6.0 | 6.0 |

As xanthan gum, Echo Gum T manufactured by Dainippon Sumitomo Pharma Co., Ltd. was used.

**Table 5**

| Formulation (g/100 mL) | Comparative Example 8 | Comparative Example 9 | Comparative Example 10 | Comparative Example 11 | Comparative Example 12 | Example 5 | Example 6 | Example 7 | Example 8 | Example 9 |
|---|---|---|---|---|---|---|---|---|---|---|
| Gatifloxaci n 3/2 | - | - | - | - | - | 0.32 | 0.32 | 0.32 | 0.32 | 0.32 |
| hydrate Xanthan gum | 0.2 | 0.2 | 0.2 | 0.2 | 0.2 | 0.2 | 0.2 | 0.2 | 0.2 | 0.2 |
| Sodium chloride | - | 0.2 | 0.7 | 0.9 | 1.75 | - | 0.2 | 0.7 | 0.9 | 1.75 |
| Hydrochloric acid | adequate amount | adequate amount | adequate amount | adequate amount | adequate amount | adequate amount | adequate amount | adequate amount | adequate amount | adequate amount |
| Sodium hydroxide | adequate amount | adequate amount | adequate amount | adequate amount | adequate amount | adequate amount | adequate amount | adequate amount | adequate amount | adequate amount |
| Purified water | adequate amount | adequate amount | adequate amount | adequate amount | adequate amount | adequate amount | adequate amount | adequate amount | adequate amount | adequate amount |
| Total amount | 100 mL | 100 mL | 100 mL | 100 mL | 100 mL | 100 mL | 100 mL | 100 mL | 100 mL | 100 mL |
| pH | 7.0 | 6.9 | 7.1 | 7.1 | 7.0 | 7.0 | 7.1 | 7.0 | 7.0 | 7.0 |

As xanthan gum, Echo Gum T manufactured by Dainippon Sumitomo Pharma Co., Ltd was used.

### Test Example 2

Effect of suppressing formation of a precipitate and reduction in viscosity by addition of sodium chloride 1. Experimental method

Appearance of each of eye drops shown in Table 4 and Table 5 was observed. In addition, 1.2 mL of each of eye drops was taken, and placed into a sample cup of a rotation viscometer (instrument used: E-type rotation viscometer: TVE-20L (roter No.1), manufactured by Toki Sangyo Co., Ltd.) and viscosity was measured. The rotation speed was 20 rpm, and temperature was 20°C.

### 2. Results

Results are shown in Table 6 and Table 7.

**Table 6**

| Formulation | Comparative Example 3 | Comparative Example 4 | Comparative Example 5 | Comparative Example 6 | Comparative Example 7 | Example 2 | Example 3 | Example 4 |
|---|---|---|---|---|---|---|---|---|
| Appearance | Clear | Clear | Clear | Clear | Precipitate | Clear | Clear | Clear |
| Viscosity (mPa·s) | 37.07 | 25.07 | 25.61 | 25.76 | - | 16.34 | 19.63 | 23.32 |

Viscosity of Comparative Example 7 was not measured because of generation of a precipitate.

**Table 7**

| Formulation | Comparative Example 8 | Comparative Example 9 | Comparative Example 10 | Comparative Example 11 | Comparative Example 12 | Example 5 | Example 6 | Example 7 | Example 8 | Example 9 |
|---|---|---|---|---|---|---|---|---|---|---|
| Appearance | Clear | Clear | Clear | Clear | Clear | Clear | Clear | Clear | Clear | Clear |
| Viscosity (mPa·s) | 29.97 | 25.15 | 25.75 | 25.7 | 25.61 | 16.18 | 17.68 | 22.2 | 23.4 | 24.39 |

As shown in Comparative Examples 4 to 6, 9 to 11, in the aqueous liquid preparations not containing gatifloxacin, sodium chloride reduced viscosity of the aqueous liquid preparation, and this reducing action did not depend on a concentration of sodium chloride.

On the other hand, as shown in Examples 2 to 4, and 5 to 9, in the aqueous liquid preparations containing gatifloxacin, sodium chloride suppressed reduction in viscosity of the aqueous liquid preparation, and this suppressing action depended on a concentration of sodium chloride.

### Examples 10 to 17

According to the formulations of Table 8, gatifloxacin eye drops were prepared by a conventional method.

**Table 8**

| Formulation (g/100 mL) | Example 10 | Example 11 | Example 12 | Example 13 | Example 14 | Example 15 | Example 16 | Example 17 |
|---|---|---|---|---|---|---|---|---|
| Gatifloxacin 3/2 hydrate | 0.2 | 0.3 | 0.3 | 0.5 | 05 | 0.8 | 1.0 | 0.1 |
| Xanthan gum | 0.2 | 0.3 | 0.5 | 0.3 | 0.5 | 0.2 | 0.2 | 1.0 |
| Sodium chloride | 0.75 | 0.9 | 0.9 | 0.9 | 0.9 | 0.9 | 0.9 | 0.5 |
| Disodium edetate | - | 0.02 | - | - | 0.02 | - | - | - |
| Sodium dihydrogen phosphate | - | - | 0.1 | - | - | 0.1 | - | - |
| Benzalkonium chloride | 0.005 | - | - | - | - | - | - | - |
| Methyl paraoxybenzoate | - | - | - | 0.026 | - | - | 0.026 | - |
| Propyl paraoxybenzoate | - | - | - | 0.014 | - | - | 0.014 | - |
| Boric acid | 0.4 | - | - | - | - | - | - | 0.8 |
| Hydrochloric acid/sodium hydroxide | adequate amount | adequate amount | adequate amount | adequate amount | adequate amount | adequate amount | adequate amount | adequate amount |
| Sterile purified water | adequate amount | adequate amount | adequate amount | adequate amount | adequate amount | adequate amount | adequate amount | adequate amount |
| Total amount | 100 mL | 100 mL | 100 mL | 100 mL | 100 mL | 100 mL | 100 mL | 100 mL |
| pH | 7.0 | 6.5 | 6.0 | 5.5 | 5.5 | 5.5 | 5.8 | 7.0 |

### Industrial Applicability

As described above, according to the present invention, by addition of xanthan gum, gatifloxacin-containing ophthalmic aqueous liquid preparations having improved intraocular penetration of gatifloxacin can be provided and, in addition, by adding sodium chloride, formation of a precipitate and reduction in viscosity in the aqueous liquid preparation can be suppressed.

## Claims

1. An aqueous liquid preparation comprising gatifloxacin or its pharmacologically acceptable salt, or a hydrate thereof, at least 0.15 w/v% of xanthan gum and at least 0.2 w/v% of sodium chloride, and having a pH of 5 to 8.

2. The aqueous liquid preparation according to claim 1, wherein the preparation contains 0.15 to 1.0 w/v% of xanthan gum.

3. The aqueous liquid preparation according to claim 1, wherein the preparation contains 0.2 to 1.8 w/v% of sodium chloride.

4. The aqueous liquid preparation according to claim 1, wherein the preparation contains 0.1 to 1.0 w/v% of gatifloxacin.

5. The aqueous liquid preparation according to any one of claims 1 to 4, which is an eye drop.

6. A method for improving intraocular penetration of gatifloxacin, which comprises incorporating at least 0.15 w/v% of xanthan gum into an aqueous liquid preparation containing gatifloxacin or its pharmacologically acceptable salt thereof, or a hydrate thereof.

7. A method for suppressing formation of a precipitate and reduction in viscosity in an aqueous liquid preparation containing gatifloxacin and xanthan gum, which comprises incorporating at least 0.2 w/v% of sodium chloride into an aqueous liquid preparation containing gatifloxacin or its pharmacologically acceptable salt thereof, or a hydrate thereof, and at least 0.15 w/v% of xanthan gum.
